# EUROPEAN PATENT SPECIFICATION

(11) **EP 4 669 206 B1**
(45) Date of publication and mention of the grant of the patent: **08.04.2026**
(21) Application number: 24706960.2
(22) Date of filing: 19.02.2024
(51) Int. Cl.: A61B 6/03, A61B 6/00

(54) **MODERATED RESCAN FOR MOTION-CORRUPTED MEDICAL IMAGING SCANS**
MODERIERTER RE-SCAN FÜR MEDIZINISCHE BILDGEBUNGSSCANS MIT BEWEGUNGSFEHLERN
RE-SCAN MODÉRÉ POUR SCANS D'IMAGERIE MEDICALE AFFECTÉS PAR MOUVEMENT

(30) Priority: 24.02.2023 US 202363448036 P
(43) Date of publication of application: 31.12.2025
(73) Proprietor: Koninklijke Philips N.V., 5656 AG Eindhoven (NL)
(72) Inventor: SCHUELKE, Christophe Michael Jean, 5656 AG Eindhoven (NL); WILD, Sebastian, 5656 AG Eindhoven (NL); KOEHLER, Thomas, 5656 AG Eindhoven (NL); BIPPUS, Rolf Dieter, 5656 AG Eindhoven (NL); BERGNER, Frank, 5656 AG Eindhoven (NL); GRASS, Michael, 5656 AG Eindhoven (NL)
(74) Representative: Philips Intellectual Property & Standards
(86) International application number: PCT/EP2024/054094
(87) International publication number: WO 2024/175515

(56) References cited:
- US-A1- 2018 350 081
- US-A1- 2021 196 219

## Description

### FIELD OF THE INVENTION

The invention relates to the field of medical imaging and, in particular, to a method and apparatus for performing a moderated rescan of motion-corrupted medical imaging scans.

### BACKGROUND OF THE INVENTION

Medical imaging systems include non-invasive examination devices that can capture detailed images of structures, tissue, fluid flow, etc., inside a body and provide the images to a user. A user, for example, a radiologist or a medical practitioner, may use images from the medical imaging system to diagnose a patient's condition.

Examples of a medical imaging systems include a Computed Tomography (CT) system, a Computed Axial Tomography (CAT) system, an interventional X-ray system, a Magnetic Resonance Imaging (MRI) system, an X-ray fluoroscopy system, a Positron Emission Tomography (PET) system, a Single Photon Emission Computed Tomography (SPECT) system, etc.

A CT imaging system provides a cross-sectional image of an object. A CT imaging system emits an X-ray towards an object, detects the X-ray that has passed through the object, and reconstructs an image by using the detected X-ray. Furthermore, the CT imaging system may represent an internal structure (e.g., organs such as kidney, lung, etc.) of the object without superimposition of other body parts in front of and/or behind the internal structure, unlike a traditional X-ray apparatus. CT imaging systems are widely used for precise diagnosis of diseases due to these advantages.

MRI imaging is a non-invasive imaging technology that produces three dimensional detailed anatomical images. MRIs employ powerful magnets which produce a strong magnetic field that forces protons in the body to align with that field. When a radiofrequency current is then pulsed through the patient, the protons are stimulated and spin out of equilibrium, straining against the pull of the magnetic field. When the radiofrequency field is turned off, the MRI sensors are able to detect the energy released, as the protons realign with the magnetic field. MRIs are based on sophisticated technology that excites and detects changes in protons found in the water that makes up living tissues to capture an image of a subject, and are widely used to accurately diagnose diseases, because it shows stereoscopic images of bones, lumbar discs, joints, nerve ligaments, etc., at desired angles. MIRs differs from a CTs in that they do not emit ionizing radiation of X-rays. Because an MRI does not use X-rays, it may be a good imaging modality when frequent imaging is required. However, some studies show that the electric and magnetic fields (EMF) generated during an MRI scan may have biological and genotoxic health implications.

Medical images obtained by the various medical imaging systems depict an object in various ways according to the type of medical imaging system and scanning method used, and a medical professional may analyze a medical image and determine the presence of an injury, disease, or abnormal health conditions in a patient.

The amount of radiation exposure from an imaging test depends on the type of imaging used and the part of the body being imaged. An average person is exposed to about 3 millisieverts (mSv) of radiation from natural sources over the course of a year. A single chest x-ray may expose the patient to about 0.1 mSv. This is about the same amount of radiation a person is exposed to naturally over the course of about 11-12 days. A CT scan of the abdomen and pelvis may expose a person to about 10 mSv, and a PET/CT scan may exposes a person to about 25 mSv of radiation. This is equal to about 8 years of average background radiation exposure. Thus, limiting a patient's exposure amount and time during medical imaging scans is a fundamental concern. While advances in hardware and in reconstruction algorithms have been successful in reducing the dose for individual scans, repeating an imaging scan may pose additional risk to a patient.

Despite the short duration of most imaging scans, patient movement may occur during imaging acquisition, and this may result in non-diagnostic scan images. In such a case, the scan must be repeated. From a workflow perspective, an easy approach is to repeat the exact same scan. This results in twice the dose of radiation for the same result.

In some cases, only a limited part of the image is corrupted by motion artifacts. In that case, it might be possible to rescan the limited part of the anatomy, thereby avoiding exposure to the rest of the anatomy. However, this requires additional planning time and will produce two distinct sets of images, both of which might be necessary for a full diagnosis, thus making diagnosis more cumbersome for the radiologist.

In the case where a rescan is necessary due to patient motion, the user therefore must choose between giving an additional dose to the patient or taking more time to complete the exam and the radiologist diagnosis.

Thus, the need (technical problem) exists for a solution to perform a rescan that limits or reduces exposure to a patient and provides the user or radiologist with a single diagnostic image.

US 2018/350081 A1 discloses a motion-gated medical imaging system. In an embodiment, data acquisition may be terminated when significant motion is detected during a scan.

US 2021/196219 A1 discloses methods and system for motion detection in position emission tomography. In an example, a CT scan of a patient may be repeated after motion is detected.

### SUMMARY OF THE INVENTION

The object of the present invention (technical solution) provides techniques for performing a rescan, such that the applied dose is minimized, the planning time of the rescan is minimal, and the result is a single diagnostic image obtained by using both the initial scan and the rescan.

The techniques may be applied to virtually any interventional radiology imaging system including, but not limited to, a Computed Tomography (CT) system, a Computed Axial Tomography (CAT) system, an interventional X-ray system, a Magnetic Resonance Imaging (MRI) system, an X-ray fluoroscopy system, a Positron Emission Tomography (PET) system, and a Single Photon Emission Computed Tomography (SPECT) system.

In one aspect of the present invention, a method of performing a rescan for motion-corrupted medical imaging scans as defined in claim 1 is provided.
Other aspects of the present invention include a corresponding medical imaging system and a non-transitory computer-readable medium as defined in claims 8 and 15, respectively.

Implementations may include one or more of the following features. The method where at least one of: detecting motion during the initial imaging scan of the patient, identifying the one or more regions of the patient's anatomy where motion artifacts are present, and determining a rescan is required in at least one of the identified one or more regions is performed by at least one of: an user, a dedicated neural network, or information from at least one sensor. The method where initiating the procedure for adapting the moderated rescan of the patient includes automatically proposing a set of moderated rescan parameters. The method where the moderated rescan is limited to image stacks where the motion artifacts are present and the image stacks from the moderated rescan are registered and stitched back together with image stacks from the initial imaging scan to form one coherent image. The method where the moderated rescan is performed with a dose of radiation lower than the initial imaging scan and generating the final image reconstruction may include a motion compensated reconstruction algorithm using data from the initial imaging scan and the moderated rescan to generate the final image reconstruction with reduced motion artifacts. The method where the moderated rescan yields redundant data and generating the final image reconstruction may include a motion compensated reconstruction algorithm using data from the initial imaging scan and the moderated rescan to generate the final image reconstruction with reduced motion artifacts. The method where the moderated rescan is limited to a reduced coverage region and the moderated rescan is performed on the reduced coverage region with a dose of radiation lower than the dose of radiation in the initial imaging scan and a resulting image, obtained from a motion compensated reconstruction using the data from the initial imaging scan and the data from the moderated rescan, is registered and stitched back into a correct location of the initial imaging scan to form one coherent image. The method where the user adjusts one or more parameters of the set of moderated rescan parameters and initiates the moderated rescan, or the user accepts the set of moderated rescan parameters and initiates the moderated rescan. The method where the set of moderated rescan parameters are automatically accepted and the moderated rescan is automatically performed directly. Implementations of the described techniques may include hardware, a method or process, or a computer tangible medium.

**In** another general aspect, a medical imaging system is provided. The medical imaging system may include a memory configured to store a plurality of instructions and processor circuitry coupled to the memory and configured to execute the plurality of instructions to: perform an initial imaging scan of a patient, detect patient motion during the initial imaging scan of the patient, identify one or more regions of the patient's anatomy where motion artifacts are present, determine a rescan is required in at least one of the identified one or more regions, initiate a procedure for adapting a moderated rescan of the patient in the at least one of the identified one or more regions where the rescan is required, perform the moderated rescan, and generate a final image reconstruction with coverage and characteristics of the initial imaging scan with reduced motion artifacts based on data from the initial imaging scan and the moderated rescan. The generating the final reconstruction comprises a motion compensated reconstruction algorithm using data of the one or more identified regions from the initial imaging scan and the moderated rescan to generate the final image reconstruction with the reduced motion artifacts.

Implementations may include one or more of the following features. The medical imaging system where at least one of: detecting motion during the initial imaging scan of the patient, identifying the one or more regions of the patient's anatomy where motion artifacts are present, and determining a rescan is required in at least one of the identified one or more regions is performed by at least one of: a user, a dedicated neural network, or information from at least one sensor. The medical imaging system where initiating the procedure for adapting the moderated rescan of the patient includes automatically proposing a set of moderated rescan parameters. The medical imaging system where the moderated rescan is limited to image stacks where the motion artifacts are present and the image stacks from the moderated rescan are registered and stitched back together with image stacks from the initial imaging scan to form one coherent image. The medical imaging system where the moderated rescan is performed with a dose of radiation lower than the initial imaging scan and generating the final image reconstruction may include a motion compensated reconstruction algorithm using data from the initial imaging scan and the moderated rescan to generate the final image reconstruction with reduced motion artifacts. The medical imaging system where the moderated rescan yields redundant data and generating the final image reconstruction may include a motion compensated reconstruction algorithm using data from the initial imaging scan and the moderated rescan to generate the final image reconstruction with reduced motion artifacts. The medical imaging system where the moderated rescan is limited to a reduced coverage region and the moderated rescan is performed on the reduced coverage region with a dose of radiation lower than the dose of radiation in the initial imaging scan and a resulting image, obtained from a motion compensated reconstruction using the data from the initial imaging scan and the data from the moderated rescan, is registered and stitched back into a correct location of the initial imaging scan to form one coherent image. The medical imaging system where the user adjusts one or more parameters of the set of moderated rescan parameters and initiates the moderated rescan, or the user accepts the set of moderated rescan parameters and initiates the moderated rescan. The medical imaging system where the set of moderated rescan parameters are automatically accepted and the moderated rescan is automatically performed directly.

In yet another general aspect, a non-transitory computer-readable medium having stored thereon instructions for causing processing circuitry to execute a process is provided. The process may include detecting patient motion during the initial imaging scan of the patient, identifying one or more regions of the patient's anatomy where motion artifacts are present, determining a rescan is required in at least one of the identified one or more regions, and initiating a procedure for adapting a moderated rescan of the patient in the at least one of the identified one or more regions where the rescan is required. The process may also include performing the moderated rescan and generating a final image reconstruction with coverage and characteristics of the initial imaging scan with reduced motion artifacts based on data from the initial imaging scan and the moderated rescan. The generating the final reconstruction comprises a motion compensated reconstruction algorithm using data of the one or more identified regions from the initial imaging scan and the moderated rescan to generate the final image reconstruction with the reduced motion artifacts.

### BRIEF DESCRIPTION OF THE DRAWINGS

In the following drawings:
Fig. 1 is a diagram of an exemplary medical imaging system according to some embodiments;
Fig. 2 illustrates an example workflow according to some embodiments; and
Fig. 3 is a flowchart of an example moderated rescan process.

### DETAILED DESCRIPTION OF EMBODIMENTS

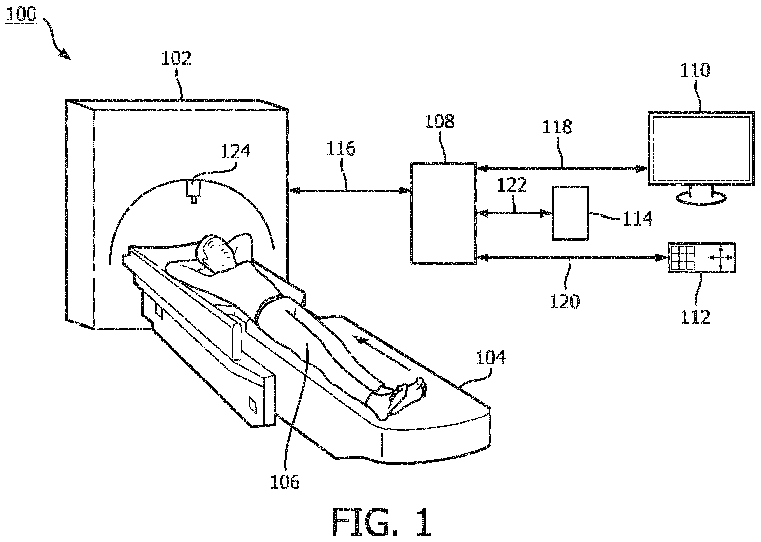

Fig. 1 is a diagram of an exemplary medical imaging system 100 according to embodiments of the present invention. The imaging system 100 includes an imaging scanning device such as a computed tomography (CT) imaging device 102, a patient support 104, and an examination subject, for example, patient 106. CT imaging device 102 uses a computerized X-ray imaging procedure in which a narrow beam of x-rays are aimed at a patient and quickly rotated around the body, producing signals that are processed by the computer to generate cross-sectional images, or "slices." Once a number of successive slices are collected by the computer, they can be digitally "stacked" together to form a three-dimensional (3D) image of the patient that allows identification of basic structures, as well as possible tumors or abnormalities.

Computer system 108 may be coupled to CT imaging device 102 via link 116. Computer system 108 generates the scan images and provides a link between the radiologist or technologist and the other components of the imaging system. Computer system 108 may be coupled with a display 110 via link 118, and may be coupled with an input device 112 via link 120. Computer system 108 used in a CT device, for example, has four basic functions: control of data acquisition, image reconstruction, storage of image data, and image display. In one or more embodiments computer system 108 may be coupled to an Artificial Intelligence (AI) engine 114 via link 122. AI engine 114 may also reside within computer system 108. In addition, imaging system 100 may include one or more sensors, for example camera 124.

In an example embodiment, when an imaging scan is performed, the one or more sensors, for example sensors/camera 124, may detect any motion of patient 106 that may have occurred during the imaging scan. The sensors/camera 124 may indicate the position of the scanner when motion is detected and provide time stamps to computer system 108 and/or AI engine 114. AI engine 114 may be a dedicated neural network, for example.

Computer system 108 may be configured to identify one or more regions of patient anatomy, where motion artifacts are present due to the motion of patient 106. In a case where computer system 108 has detected motion and identified one or more regions of the anatomy of patient 106 where motion artifacts are present, computer system 108 may determine whether a rescan is required in the one or more regions of patient anatomy. That is, computer system 108 may determine whether the motion by patient 106 renders a non-diagnostic scan image. Computer system 108 may receive input from a user, sensors/camera 124, and AI engine 114, either alone or in any combination.

Once computer system 108 determines a rescan is required, a procedure for adapting a moderated rescan of the patient in the at least one of the identified regions where the rescan is required is initiated and a moderated rescan is performed. Once the moderated rescan is performed, computer system 108 generates a final image reconstruction with coverage and characteristics of the initial imaging scan and reduced motion artifacts based on data from the initial imaging scan and the moderated rescan.

In an example embodiment, one or more of: detecting motion during the initial imaging scan of the patient, identifying the one or more regions of the patient's anatomy where motion artifacts are present, and determining a rescan is required in at least one of the identified one or more regions may be performed by a user with or without information from at least one sensor. For example, a user such as a radiologist may observe the imaging scan, identify a region that contains motion artifacts, and determine whether a rescan is necessary.

In another embodiment, one or more of: detecting motion during the initial imaging scan of the patient, identifying the one or more regions of the patient's anatomy where motion artifacts are present, and determining a rescan is required in at least one of the identified one or more regions may be performed with AI methods such as a dedicated neural network. The AI methods may include receiving data from one or more sensors such as sensors/camera 124 as an input. AI engine 114, may be a neural network coupled with or embedded in computer system 108. For example, a dedicated neural network may view the scan image to access the presence of motion artifacts and return a bounding box containing the region with motion artifacts.

While Fig. 1 illustrates a medical imaging system using CT imaging device 102, it should be understood that the techniques may be applied to a number of medical imaging devices/systems including: a CAT system, an interventional X-ray system, an MRI system, an X-ray fluoroscopy system, a PET system, and a SPECT system.

Certain medical imaging systems emit radiation in the form of an X-ray, and other medical imaging systems emit electromagnetic fields, which may be referred to as EMF. However, in each of the above identified medical imaging systems, the techniques for performing a moderated rescan reduces the patient's exposure to the emitted source.

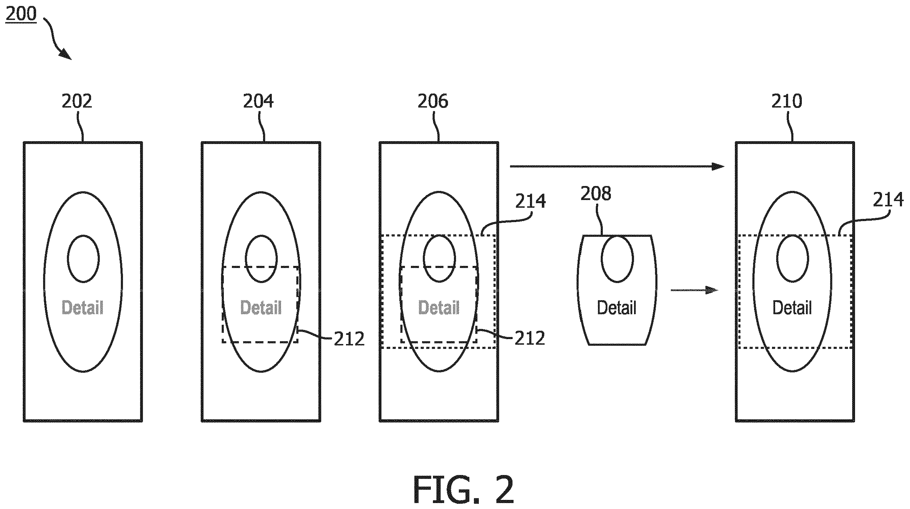

Fig. 2 illustrates an example workflow according to some embodiments. After an initial scan, at 202 motion is detected by at least one of a user/operator, neural network, and external sensor, either alone or in combination. The term "detail" is blurred to illustrate an area or region of a patient's anatomy where the quality of a scan image is diminished due to a patient's motion that occurred during the imaging scan. In other words, the blurred term "detail" represents a motion artifact in the image.

As illustrated in Fig. 2, motion artifact localization is performed at 204. Namely, an area or region of a patient's anatomy, where motion artifacts are present and a rescan is required, is identified and localized. The localized region is identified by the dashed line 212. Localization may be performed by at least one of a user/operator, neural network, and external sensor, either alone or in combination. At 206 a procedure for adapting a moderated rescan is initiated. The moderated rescan may be performed with a reduced dose. The reduced dose may be achieved by limiting or reducing the emitted source (for example, the X-ray radiation or EMF may be reduced), limiting or reducing the coverage area, or a combination of reducing the emitted source and reducing the coverage area.

In one embodiment, the procedure for adapting a moderated rescan 206 may include automatically proposing a set of moderated rescan parameters. In an embodiment, a set of moderated rescan parameters may be presented to the user, for example a radiologist, and the user may validate or edit each parameter in the set of parameters. The set of parameters may be based on the spatial extent and severity of the motion artifacts, parameters of the initial scan, and current or updated position of the patient. The set of parameters may be chosen to minimize the coverage region and dose of the moderated rescan, while ensuring that the data from the initial scan and from the moderated rescan may be used to form a single, coherent diagnostic image.

In an alternative embodiment, the set of moderated rescan parameters may be automatically accepted and the moderated rescan automatically performed without delay. This may be necessary when a scan is performed with contrast. Contrast, which is typically a fluid with good detection properties in the selected modality but also good biocompatibility, may be used to emphasize structures, blood vessels, internal organs, and the like, and the contrast material may be given to a patient orally, by injection, or other means. The type of contrast material may vary depending on the type of imaging and part of the anatomy being examined. For example, iodine and barium are often used with CT imaging, and gadolinium is often used with MRI imaging. The half-life of the respective contrast agents varies with some being cleared as rapidly as 45 seconds. Thus, an implementation where the set of moderated rescan parameters may be automatically accepted and the moderated rescan automatically performed without delay may be imperative when performing an imaging scan of a patient with contrast.

As shown at 206, the region identified by dashed line 214 is selected as the region for performing the moderated rescan. Axial scans with reduced collimation may require a segmented acquisition of several neighboring stacks in order to achieve the desired coverage. In helical scans with a large coverage region, motion artifacts might also be present in a limited region of the image. If the region is small enough, an axial rescan at that location might be sufficient. If not, a helix shorter than the initial one may be sufficient to image the region. Accordingly, the moderated rescan region 214 may be dependent on the type of scan and the localization artifact 212.

A moderated rescan is performed on the region 214, and at 208 moderated rescan reconstruction is performed. Reconstruction 208 may be performed using data acquired in the moderated rescan. Reconstruction 208 may be performed using data acquired from the moderated rescan and data from the initial scan for a motion-compensated reconstruction (MCR). In either case, the reconstructed image 208 reduces or eliminates the motion artifacts, as illustrated by the clarity of the term "detail" at 208. At 210 a final image is generated. The final image is generated by stitching images from the initial scan and moderated rescan, resulting in a single coherent image.

In one embodiment of the present invention, the moderated rescan may be limited to image stacks where the motion artifacts are present, thus reducing the patient's exposure as compared to the initial scan. The image stacks from the moderated rescan are registered and stitched back together with image stacks from the initial imaging scan to form a single coherent image. Thus, the patient's exposure is reduced due to the rescan being limited to the region with motion artifacts. In addition, there is neither additional planning time nor two distinct sets of images necessary for a full diagnosis.

In one embodiment of the present invention, MCR techniques may be used. Typically, MCR techniques require a certain amount of data redundancy to correct for motion that may have occurred during the initial scan. As data redundancy is equivalent to higher dose, it is not desirable to use this type of scan protocol by default. However, a rescan with low dose may be sufficient to provide the necessary data redundancy. Thus, in one embodiment, an MCR algorithm uses the data from the initial scan and data from the moderated rescan scan to generate the scan image.

For example, in the case of a helical scan, the very low dose may be obtained by using a very large pitch, faster rotation time, or by reducing the tube current. The MCR using the data from the initial scan and data from the moderated rescan scan may provide images with reduced or eliminated motion artifacts. One specific implementation of this approach is to use the data from the moderated rescan as a motion free reference state for the motion compensated reconstruction (MCR). This avoids the usual problem in MCR where a registration of image sets that both have motion artifacts is required. This approach may be beneficial when a contrast agent is used, as the distribution of the contrast agent is more consistent.

In another embodiment of the present invention, both the coverage and the dose of the moderated rescan may be reduced. For example, the emitted dose may be reduced to a strict minimum, and the MCR may be performed on a reduced region minimizing the reconstruction time. The moderated rescan image may then be stitched together with the region on the initial scan that does not contain motion artifacts.

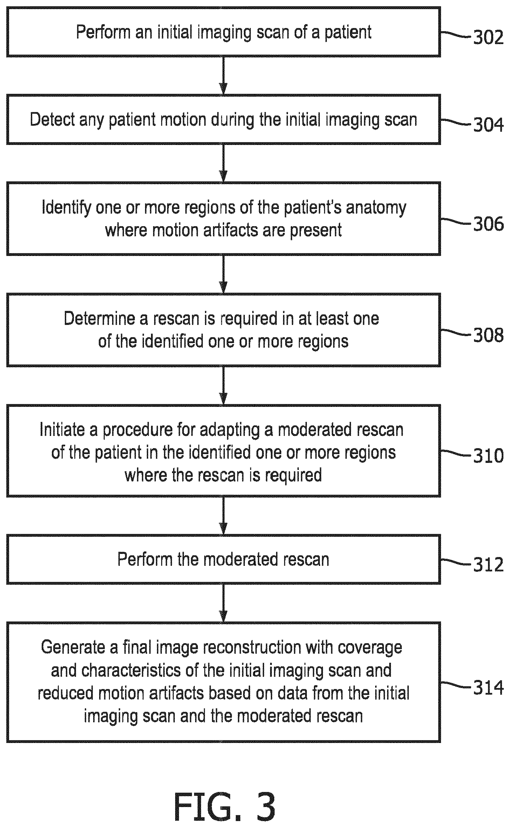

Fig. 3 is a flowchart of an example rescan process 300. In some embodiments, one or more process blocks of Fig. 3 may be performed by a single device.

As shown in Fig. 3, process 300 may include performing an initial medical imaging scan of a patient at 302. For example, an initial medical imaging scan of a patient may be performed. Process 300 may include detecting any patient motion that may have occurred during the initial imaging scan at 304. For example, a user, computer system, neural network, and/or one or more sensors may detect any patient motion during the initial imaging scan, as described above. As further shown in Fig. 3, process 300 may include identifying one or more regions of the patient's anatomy where motion artifacts are present at 306. For example, a user, computer system, neural network, and/or one or more sensors may identify one or more regions of the patient's anatomy where motion artifacts are present due to motion of the patient during the initial imaging scan. As also shown in Fig. 3, process 300 may include determining a rescan is required in at least one of the identified one or more regions at 308. For example, a user, computer system, neural network, and/or one or more sensors may determine a rescan is required in at one or more regions of the patient's anatomy, as described above. As further shown in Fig. 3, process 300 may include initiating a procedure for adapting a moderated rescan of the patient in the regions where the rescan is required at 310. For example, a user, computer system, and/or neural network may initiate a procedure for adapting a moderated rescan of the patient in the identified regions where the rescan is required. As also shown in Fig. 3, process 300 may include performing the moderated rescan at 312. Process 300 may include generating a final image reconstruction with coverage and characteristics of the initial imaging scan and reduced or eliminated motion artifacts based on data from the initial imaging scan and the moderated rescan at 314.

Process 300 may include additional implementations, such as any single implementation or any combination of implementations described below and/or in connection with one or more other processes described elsewhere herein.

Although Fig. 3 shows example blocks of process 300, in some implementations, process 300 may include additional blocks, fewer blocks, different blocks, or differently arranged blocks than those depicted in Fig. 3. Additionally, or alternatively, two or more of the blocks of process 300 may be performed in parallel.

While aspects have been illustrated and described in detail in the drawings and foregoing description, such illustrations and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single processor, device or other unit may fulfill the functions of several items recited in the claims. The mere fact that certain measures are recited in mutually different dependent claims does not indicate that a combination of these measures cannot be used to advantage.

Operations like acquiring, determining, obtaining, outputting, providing, store or storing, calculating, simulating, receiving, warning, and stopping can be implemented as program code means of a computer program and/or as dedicated hardware.

A computer program may be stored and/or distributed on a suitable medium, such as an optical storage medium or a solid-state medium, supplied together with or as part of other hardware, but may also be distributed in other forms, such as via the Internet or other wired or wireless telecommunication systems.

## Claims

1. A method of performing a rescan for motion-corrupted medical imaging scans, comprising:
performing (302) an initial imaging scan of a patient;
detecting (304) any patient motion during the initial imaging scan;
identifying (306) one or more regions (212, 214) of the patient's anatomy where motion artifacts are present;
determining (308) whether a rescan is required in at least one of the identified one or more regions;
initiating (310) a procedure for adapting a moderated rescan of the patient in the at least one of the identified one or more regions where the rescan is required, the moderated scan being performed by limiting or reducing a tube current, a coverage area or a combination of both;
performing (312) the moderated rescan; and
generating (314) a final image reconstruction with coverage and characteristics of the initial imaging scan with reduced motion artifacts, the final image reconstruction being based on data from the initial imaging scan and the moderated rescan,
wherein the generating the final reconstruction comprises a motion compensated reconstruction algorithm (208) using data of the one or more identified regions from the initial imaging scan and the moderated rescan to generate the final image reconstruction with the reduced motion artifacts.

2. The method according to claim 1, wherein at least one of: detecting motion during the initial imaging scan of the patient, identifying the one or more regions of the patient's anatomy where motion artifacts are present, and determining a rescan is required in at least one of the identified one or more regions is performed by at least one of: a user, a dedicated neural network, and information from at least one sensor.

3. The method according to claim 2, wherein initiating the procedure for adapting the moderated rescan of the patient further comprises automatically proposing a set of moderated rescan parameters.

4. The method according to claim 3, wherein the moderated rescan is performed with a dose of radiation lower than the initial imaging scan.

5. The method according to claim 3, wherein the moderated rescan is limited to a reduced coverage region, and the moderated rescan is performed on the reduced coverage region with a dose of radiation lower than the dose of radiation in the initial imaging scan and a resulting image, obtained from a motion compensated reconstruction using the data from the initial imaging scan and the data from the moderated rescan, is registered and stitched into a correct location of the initial imaging scan to form one coherent image.

6. The method according to claim 3, wherein one or more parameters of the set of moderated rescan parameters is adjusted or the set of moderated rescan parameters is accepted without an adjustment.

7. The method according to claim 3, wherein the set of moderated rescan parameters are automatically accepted and the moderated rescan is performed automatically.

8. A medical imaging system (100), comprising:
a memory configured to store a plurality of instructions; and
processor circuitry coupled to the memory and configured to execute the plurality of instructions to:
perform (302) an initial imaging scan of a patient;
detect (304) patient motion during the initial imaging scan of the patient;
identify (306) one or more regions of the patient's anatomy where motion artifacts are present;
determine (308) whether a rescan is required in at least one of the identified one or more regions;
initiate (310) a procedure for adapting a moderated rescan of the patient in the at least one of the identified one or more regions where the rescan is required, the moderated scan being performed by limiting or reducing a tube current, a coverage area or a combination of both;
perform (312) the moderated rescan; and
generate (314) a final image reconstruction with coverage and characteristics of the initial imaging scan with reduced motion artifacts, the final image reconstruction being based on data from the initial imaging scan and the moderated rescan,
wherein generating the final image reconstruction comprises a motion compensated reconstruction algorithm using data from the initial imaging scan and the moderated rescan to generate the final image reconstruction with reduced motion artifacts.

9. The medical imaging system according to claim 8, wherein at least one of: detecting motion during the initial imaging scan of the patient, identifying the one or more regions of the patient's anatomy where motion artifacts are present, and determining a rescan is required in at least one of the identified one or more regions is performed by at least one of: a user, a dedicated neural network, or information from at least one sensor.

10. The medical imaging system according to claim 9, wherein initiating the procedure for adapting the moderated rescan of the patient further comprises automatically proposing a set of moderated rescan parameters.

11. The medical imaging system according to claim 10, wherein the moderated rescan is performed with a dose of radiation lower than the initial imaging scan.

12. The medical imaging system according to claim 10, wherein the moderated rescan is limited to a reduced coverage region, and the moderated rescan is performed on the reduced coverage region with a dose of radiation lower than the dose of radiation in the initial imaging scan, and a resulting image, obtained from a motion compensated reconstruction using the data from the initial imaging scan and the data from the moderated rescan, is registered and stitched into a correct location of the initial imaging scan to form one coherent image.

13. The medical imaging system according to claim 10, wherein one or more parameters of the set of moderated rescan parameters is adjusted or the set of moderated rescan parameters is accepted without an adjustment.

14. The medical imaging system according to claim 10, wherein the set of moderated rescan parameters are automatically accepted and the moderated rescan is performed automatically.

15. A non-transitory computer-readable medium having stored thereon instructions for causing processing circuitry to execute a process (300), the process comprising:
performing (302) an initial imaging scan of a patient;
detecting (304) patient motion during the initial imaging scan of the patient;
identifying (306) one or more regions of the patient's anatomy where motion artifacts are present;
determining (308) whether a rescan is required in at least one of the identified one or more regions;
initiating (310) a procedure for adapting a moderated rescan of the patient in the at least one of the identified one or more regions where the rescan is required, the moderated scan being performed by limiting or reducing a tube current, a coverage area or a combination of both;
performing (312) the moderated rescan; and
generating (314) a final image reconstruction with coverage and characteristics of the initial imaging scan with reduced motion artifacts, the final image reconstruction being based on data from the initial imaging scan and the moderated rescan,
wherein generating the final image reconstruction comprises a motion compensated reconstruction algorithm using data from the initial imaging scan and the moderated rescan to generate the final image reconstruction with reduced motion artifacts.

## Patentansprüche

1. Verfahren zum Durchführen eines erneuten Scans für bewegungsgestörte medizinische Bildgebungs-Scans, umfassend:
Durchführen (302) eines anfänglichen Bildgebungs-Scans eines Patienten;
Erfassen (304) jeglicher Patientenbewegung während des anfänglichen Bildgebungs-Scans;
Identifizieren (306) eines oder mehrerer Bereiche (212, 214) der Anatomie des Patienten, in denen Bewegungsartefakte vorhanden sind;
Bestimmen (308), ob in mindestens einem von einem oder mehreren identifizierten Bereichen ein erneuter Scan erforderlich ist;
Initiieren (310) einer Vorgehensweise zum Anpassen eines moderierten erneuten Scans des Patienten in dem mindestens einen des einen oder der mehreren identifizierten Bereiche, in denen der erneute Scan erforderlich ist, wobei der moderierte Scan durch Begrenzen oder Reduzieren eines Röhrenstroms, einer Abdeckungsfläche oder einer Kombination aus beidem durchgeführt wird;
Durchführen (312) des moderierten erneuten Scans; und
Erzeugen (314) einer endgültigen Bildrekonstruktion mit Abdeckung und Merkmalen des anfänglichen Bildgebungs-Scans mit reduzierten Bewegungsartefakten, wobei die endgültige Bildrekonstruktion auf Daten aus dem anfänglichen Bildgebungs-Scan und dem moderierten erneuten Scan basiert.
wobei das Erzeugen der endgültigen Rekonstruktion einen bewegungskompensierten Rekonstruktionsalgorithmus (208) umfasst, der Daten des einen oder der mehreren identifizierten Bereiche aus dem anfänglichen Bildgebungs-Scan und dem moderierten erneuten Scan verwendet, um die endgültige Bildrekonstruktion mit den reduzierten Bewegungsartefakten zu erzeugen.

2. Verfahren nach Anspruch 1, wobei mindestens eines von: Erfassen von Bewegung während des anfänglichen Bildgebungs-Scans des Patienten, Identifizieren des einen oder der mehreren Bereiche der Anatomie des Patienten, in denen Bewegungsartefakte vorhanden sind, und Bestimmen, ob ein erneuter Scan in mindestens einem oder mehreren identifizierten Bereichen erforderlich ist, durchgeführt wird durch mindestens eines von: einem Benutzer, einem dedizierten neuronalen Netzwerk und Informationen aus mindestens einem Sensor.

3. Verfahren nach Anspruch 2, wobei das Initiieren der Vorgehensweise zum Anpassen des moderierten erneuten Scans des Patienten weiter das automatische Vorschlagen eines Satzes moderierter Parameter für erneuten Scan umfasst.

4. Verfahren nach Anspruch 3, wobei der moderierte erneute Scan mit einer Strahlendosis durchgeführt wird, die niedriger ist als die des anfänglichen Bildgebungs-Scans.

5. Verfahren nach Anspruch 3, wobei der moderierte erneute Scan auf einen reduzierten Abdeckungsbereich beschränkt ist und der moderierte erneute Scan auf dem reduzierten Abdeckungsbereich mit einer Strahlendosis durchgeführt wird, die niedriger ist als die Strahlendosis bei dem anfänglichen Bildgebungs-Scan, und ein resultierendes Bild, das aus einer bewegungskompensierten Rekonstruktion unter Verwendung der Daten aus dem anfänglichen Bildgebungs-Scan und der Daten aus dem moderierten erneuten Scan gewonnen wird, aufgezeichnet und an einer korrekten Position des anfänglichen Bildgebungs-Scans zusammengefügt wird, um ein zusammenhängendes Bild zu bilden.

6. Verfahren nach Anspruch 3, wobei ein oder mehrere Parameter des Satzes moderierter Parameter für erneuten Scan eingestellt werden, oder der Satz moderierter Parameter für erneuten Scan ohne Einstellung akzeptiert wird.

7. Verfahren nach Anspruch 3, wobei der Satz moderierter Parameter für erneuten Scan automatisch akzeptiert wird und der moderierte erneute Scan automatisch durchgeführt wird.

8. Medizinisches Bildgebungssystem (100), umfassend:
einen Speicher, der zum Speichern einer Vielzahl von Anweisungen konfiguriert ist; und
Prozessorschaltungsanordnungen, die mit dem Speicher gekoppelt und dazu konfiguriert sind, die Vielzahl von Anweisungen auszuführen zum:
Durchführen (302) eines anfänglichen Bildgebungs-Scans des Patienten;
Erfassen (304) von Patientenbewegung während des anfänglichen Bildgebungs-Scans des Patienten;
Identifizieren (306) eines oder mehrerer Bereiche der Anatomie des Patienten, in denen Bewegungsartefakte vorhanden sind;
Bestimmen (308), ob in mindestens einem von einem oder mehreren identifizierten Bereichen ein erneuter Scan erforderlich ist;
Initiieren (310) einer Vorgehensweise zum Anpassen eines moderierten erneuten Scans des Patienten in dem mindestens einen des einen oder der mehreren identifizierten Bereiche, in denen der erneute Scan erforderlich ist, wobei der moderierte Scan durch Begrenzen oder Reduzieren eines Röhrenstroms, einer Abdeckungsfläche oder einer Kombination aus beidem durchgeführt wird;
Durchführen (312) des moderierten erneuten Scans; und
Erzeugen (314) einer endgültigen Bildrekonstruktion mit Abdeckung und Merkmalen des anfänglichen Bildgebungs-Scans mit reduzierten Bewegungsartefakten, wobei die endgültige Bildrekonstruktion auf Daten aus dem anfänglichen Bildgebungs-Scan und dem moderierten erneuten Scan basiert.
wobei das Erzeugen der endgültigen Bildrekonstruktion einen bewegungskompensierten Rekonstruktionsalgorithmus umfasst, der Daten aus dem anfänglichen Bildgebungs-Scan und dem moderierten erneuten Scan verwendet, um die endgültige Bildrekonstruktion mit reduzierten Bewegungsartefakten zu erzeugen.

9. Medizinisches Bildgebungssystem nach Anspruch 8, wobei mindestens eines von: Erfassen von Bewegung während des anfänglichen Bildgebungs-Scans des Patienten, Identifizieren des einen oder der mehreren Bereiche der Anatomie des Patienten, in denen Bewegungsartefakte vorhanden sind, und Bestimmen, ob ein erneuter Scan in mindestens einem des einen oder der mehreren identifizierten Bereiche erforderlich ist, durchgeführt wird durch mindestens eines von: einem Benutzer, einem dedizierten neuronalen Netzwerk oder Informationen aus mindestens einem Sensor.

10. Medizinisches Bildgebungssystem nach Anspruch 9, wobei das Initiieren der Vorgehensweise zum Anpassen des moderierten erneuten Scans des Patienten weiter das automatische Vorschlagen eines Satzes moderierter Parameter für erneuten Scan umfasst.

11. Medizinisches Bildgebungssystem nach Anspruch 10, wobei der moderierte erneute Scan mit einer Strahlendosis durchgeführt wird, die niedriger ist als die des anfänglichen Bildgebungs-Scans.

12. Medizinisches Bildgebungssystem nach Anspruch 10, wobei der moderierte erneute Scan auf einen reduzierten Abdeckungsbereich beschränkt ist und der moderierte erneute Scan auf dem reduzierten Abdeckungsbereich mit einer Strahlendosis durchgeführt wird, die niedriger ist als die Strahlendosis bei dem anfänglichen Bildgebungs-Scan, und ein resultierendes Bild, das aus einer bewegungskompensierten Rekonstruktion unter Verwendung der Daten aus dem anfänglichen Bildgebungs-Scan und der Daten aus dem moderierten erneuten Scan gewonnen wird, aufgezeichnet und an einer korrekten Position des anfänglichen Bildgebungs-Scans zusammengefügt wird, um eines zusammenhängende Bild zu bilden.

13. Medizinisches Bildgebungssystem nach Anspruch 10, wobei ein oder mehrere Parameter des Satzes moderierter Parameter für erneuten Scan eingestellt werden, oder der Satz moderierter Parameter für erneuten Scan ohne Einstellungen akzeptiert wird.

14. Medizinisches Bildgebungssystem nach Anspruch 10, wobei der Satz moderierter Parameter für erneuten Scan automatisch akzeptiert wird und der moderierte erneute Scan automatisch durchgeführt wird.

15. Nichtflüchtiges computerlesbares Medium, auf dem Anweisungen zum Veranlassen einer Verarbeitungsschaltung gespeichert sind, einen Prozess (300) auszuführen, wobei der Prozess umfasst:
Durchführen (302) eines anfänglichen Bildgebungs-Scans eines Patienten;
Erfassen (304) von Patientenbewegung während des anfänglichen Bildgebungs-Scans des Patienten;
Identifizieren (306) eines oder mehrerer Bereiche der Anatomie des Patienten, in denen Bewegungsartefakte vorhanden sind;
Bestimmen (308), ob in mindestens einem von einem oder mehreren identifizierten Bereichen ein erneuter Scan erforderlich ist;
Initiieren (310) einer Vorgehensweise zum Anpassen eines moderierten erneuten Scans des Patienten in dem mindestens einen des einen oder der mehreren identifizierten Bereiche, in denen der erneute Scan erforderlich ist, wobei der moderierte Scan durch Begrenzen oder Reduzieren eines Röhrenstroms, einer Abdeckungsfläche oder einer Kombination aus beidem durchgeführt wird;
Durchführen (312) des moderierten erneuten Scans; und
Erzeugen (314) einer endgültigen Bildrekonstruktion mit Abdeckung und Merkmalen des anfänglichen Bildgebungs-Scans mit reduzierten Bewegungsartefakten, wobei die endgültige Bildrekonstruktion auf Daten aus dem anfänglichen Bildgebungs-Scan und dem moderierten erneuten Scan basiert.
wobei das Erzeugen der endgültigen Bildrekonstruktion einen bewegungskompensierten Rekonstruktionsalgorithmus umfasst, der Daten aus dem anfänglichen Bildgebungs-Scan und dem moderierten erneuten Scan verwendet, um die endgültige Bildrekonstruktion mit reduzierten Bewegungsartefakten zu erzeugen.

## Revendications

1. Procédé de réalisation d'un rebalayage pour des balayages d'imagerie médicale altérées par le mouvement, comprenant :
la réalisation (302) d'un balayage d'imagerie initial d'un patient ;
la détection (304) de n'importe quel mouvement du patient pendant le balayage initial ;
l'identification (306) d'une ou de plusieurs régions (212, 214) de l'anatomie du patient dans lesquelles des artefacts de mouvement sont présents ;
la détermination (308) pour savoir si un rebalayage est nécessaire dans au moins une des une ou plusieurs régions identifiées ;
le déclenchement (310) d'une procédure d'adaptation d'un rebalayage modéré du patient dans la au moins une des une ou plusieurs régions identifiées dans lesquelles le rebalayage est requis, le balayage modéré étant réalisé en limitant ou en réduisant un courant de tube, une zone de couverture ou une combinaison des deux ;
la réalisation (312) du rebalayage modéré ; et
la génération (314) d'une reconstruction d'image finale avec une couverture et des caractéristiques du balayage d'imagerie initial avec des artefacts de mouvement réduits, la reconstruction d'image finale étant basée sur des données du balayage d'imagerie initial et du rebalayage modéré,
dans lequel la génération de la reconstruction finale comprend un algorithme de reconstruction compensée en mouvement (208) utilisant des données des une ou plusieurs régions identifiées à partir du balayage d'imagerie initial et du rebalayage modéré pour générer la reconstruction d'image finale avec les artefacts de mouvement réduits.

2. Procédé selon la revendication 1, dans lequel au moins une : de la détection d'un mouvement pendant le balayage initial du patient, de l'identification des une ou plusieurs régions de l'anatomie du patient dans lesquelles des artefacts de mouvement sont présents, et de la détermination qu'un rebalayage est nécessaire dans au moins une des une ou plusieurs régions identifiées, est réalisée par au moins un : d'un utilisateur, d'un réseau neuronal dédié et d'informations provenant d'au moins un capteur.

3. Procédé selon la revendication 2, dans lequel le déclenchement de la procédure d'adaptation du rebalayage modéré du patient comprend en outre la proposition automatique d'un ensemble de paramètres de rebalayage modéré.

4. Procédé selon la revendication 3, dans lequel le rebalayage modéré est réalisé avec une dose de rayonnement inférieure à celle du balayage d'imagerie initial.

5. Procédé selon la revendication 3, dans lequel le rebalayage modéré est limité à une région de couverture réduite et le rebalayage modéré est réalisé sur la région de couverture réduite avec une dose de rayonnement inférieure à la dose de rayonnement du balayage d'imagerie initial et une image résultante, obtenue à partir d'une reconstruction compensée en mouvement utilisant les données du balayage d'imagerie initial et les données du rebalayage modéré, est enregistrée et assemblée à un emplacement correct du balayage d'imagerie initial pour former une image cohérente.

6. Procédé selon la revendication 3, dans lequel un ou plusieurs paramètres de l'ensemble de paramètres de rebalayage modéré sont ajustés ou l'ensemble de paramètres de rebalayage modéré est accepté sans ajustement.

7. Procédé selon la revendication 3, dans lequel l'ensemble de paramètres de rebalayage modéré est automatiquement accepté et le rebalayage modéré est réalisé automatiquement.

8. Système d'imagerie médicale (100), comprenant :
une mémoire configurée pour stocker une pluralité d'instructions ; et
un ensemble de circuits de processeur couplé à la mémoire et configuré pour exécuter la pluralité d'instructions pour :
réaliser (302) un balayage d'imagerie initial d'un patient ;
détecter (304) un mouvement du patient pendant le balayage d'imagerie initial du patient ;
identifier (306) une ou plusieurs régions de l'anatomie du patient dans lesquelles des artefacts de mouvement sont présents ;
déterminer (308) si un rebalayage est nécessaire dans au moins une des une ou plusieurs régions identifiées ;
déclencher (310) une procédure d'adaptation d'un rebalayage modéré du patient dans la au moins une des une ou plusieurs régions identifiées dans lesquelles le rebalayage est nécessaire, le balayage modéré étant réalisé en limitant ou en réduisant un courant de tube, une zone de couverture ou une combinaison des deux ;
réaliser (312) le rebalayage modéré ; et
générer (314) une reconstruction d'image finale avec une couverture et des caractéristiques du balayage d'imagerie initial avec des artefacts de mouvement réduits, la reconstruction d'image finale étant basée sur des données du balayage d'imagerie initial et du rebalayage modéré,
dans lequel la génération de la reconstruction d'image finale comprend un algorithme de reconstruction compensée par le mouvement utilisant des données du balayage d'imagerie initial et du rebalayage modéré pour générer la reconstruction d'image finale avec des artefacts de mouvement réduits.

9. Système d'imagerie médicale selon la revendication 8, dans lequel au moins une : de la détection du mouvement pendant le balayage initial du patient, de l'identification des une ou plusieurs régions de l'anatomie du patient dans lesquelles des artefacts de mouvement sont présents, et de la détermination qu'un rebalayage est nécessaire dans au moins une des une ou plusieurs régions identifiées, est réalisée par au moins un d'un utilisateur, d'un réseau neuronal dédié ou d'informations provenant d'au moins un capteur.

10. Système d'imagerie médicale selon la revendication 9, dans lequel le déclenchement de la procédure d'adaptation du rebalayage modéré du patient comprend en outre la proposition automatique d'un ensemble de paramètres de rebalayage modéré.

11. Système d'imagerie médicale selon la revendication 10, dans lequel le rebalayage modéré est réalisé avec une dose de rayonnement inférieure à celle du balayage d'imagerie initial.

12. Système d'imagerie médicale selon la revendication 10, dans lequel le rebalayage modéré est limité à une région de couverture réduite et le rebalayage modéré est réalisé sur la région de couverture réduite avec une dose de rayonnement inférieure à la dose de rayonnement du balayage d'imagerie initial, et une image résultante, obtenue à partir d'une reconstruction compensée en mouvement utilisant les données du balayage d'imagerie initial et les données du rebalayage modéré, est enregistrée et assemblée à un emplacement correct du balayage d'imagerie initial pour former une image cohérente.

13. Système d'imagerie médicale selon la revendication 10, dans lequel un ou plusieurs paramètres de l'ensemble de paramètres de rebalayage modéré sont ajustés ou l'ensemble de paramètres de rebalayage modéré est accepté sans ajustement.

14. Système d'imagerie médicale selon la revendication 10, dans lequel l'ensemble de paramètres de rebalayage modéré est automatiquement accepté et le rebalayage modéré est réalisé automatiquement.

15. Support non transitoire lisible par ordinateur sur lequel sont stockées des instructions pour amener un ensemble de circuits de traitement à exécuter un processus (300), le processus comprenant :
la réalisation (302) d'un balayage d'imagerie initial d'un patient ;
la détection (304) d'un mouvement du patient pendant le balayage d'imagerie initial du patient ;
l'identification (306) d'une ou de plusieurs régions de l'anatomie du patient dans lesquelles des artefacts de mouvement sont présents ;
la détermination (308) pour savoir si un rebalayage est nécessaire dans au moins une des une ou plusieurs régions identifiées ;
le déclenchement (310) d'une procédure d'adaptation d'un rebalayage modéré du patient dans la au moins une des une ou plusieurs régions identifiées dans lesquelles le rebalayage est requis, le balayage modéré étant réalisé en limitant ou en réduisant un courant de tube, une zone de couverture ou une combinaison des deux ;
la réalisation (312) du rebalayage modéré ; et
la génération (314) d'une reconstruction d'image finale avec une couverture et des caractéristiques du balayage d'imagerie initial avec des artefacts de mouvement réduits, la reconstruction d'image finale étant basée sur des données du balayage d'imagerie initial et du rebalayage modéré,
dans lequel la génération de la reconstruction finale comprend un algorithme de reconstruction compensée en mouvement utilisant des données du balayage d'imagerie initial et du rebalayage modéré pour générer la reconstruction d'image finale avec les artefacts de mouvement réduits.
